# EUROPEAN PATENT SPECIFICATION

(11) **EP 1 354 577 B1**
(45) Date of publication and mention of the grant of the patent: **02.06.2010**
(21) Application number: 03007653.3
(22) Date of filing: 03.04.2003
(51) Int. Cl.: A61G 9/02

(54) **Washing and disinfecting machine comprising an automatic rotating mechanism for a support for sanitary devices**
Wasch- und Desinfektionsmaschine mit einer automatischen Drehvorrichtung für einen Halter für sanitäre Einrichtungen
Machine à laver et désinfecter comprenant un dispositif de pivotement d'un support pour ustensiles sanitaires

(30) Priority: 19.04.2002 IT VR20020026
(43) Date of publication of application: 22.10.2003
(73) Proprietor: AT-OS S.r.l., 37030 Colognola al Colli (Verona) (IT)
(72) Inventor: Lorenzini, Paolo, 37123 Verona (IT)
(74) Representative: Savi, Alberto

(56) References cited:
- CH-A- 358 548
- DE-A- 3 628 793
- FR-A- 735 453
- FR-A- 2 068 047
- GB-A- 1 036 612
- US-A- 3 645 283
- US-A- 4 759 085

## Description

The present invention is directed to a washing-disinfecting machine as defined in the preamble of claim 1.

According to the invention the washing-disinfecting machine includes an automatic moving mechanism for the hooking support of hospital auxiliary articles such as bed-pans, urinals and the like in the inside of said washing-disinfecting machine.

This mechanism permits a rotating movement of the hooking support of bed-pans, urinals and the like in order to reduce the number of operations needed to insert the said sanitary articles, in particular the slipper-shaped bed-pans, in the washing-disinfecting machines. In this way, it is possible to obtain an automatic rotation of the hooking support that supports the sanitary articles in question so that these objects are positioned correctly before closing the washing chamber and starting the washing and disinfecting process.

The present system makes the work for hospital attendants and nurses easier than the known solutions. It is sufficient for the attendant to insert the bed-pan or urinal in the machine. The attendant does not need to rotate the bed-pan. The number of the operations to be performed for the insertion of the bed-pan is thus reduced and this is advantageous because each operation to be performed for the treatment of hospital sanitary articles such as bed-pans and the like increases the risk of a biological contamination.

As is known, hospital auxiliary sanitary articles such as bed-pans and urinals are used in bed by the patients to collect their organic rejections.

These articles must often be emptied, cleaned and disinfected by the hospital attendants and nurses for their reutilization.

When handling the auxiliary sanitary articles containing liquid or solid rejections, the hospital attendants must wear protecting gloves such as one-use gloves because the organic rejections of the patients can contain bacteria and provoke a biological contamination or infection.

The prior art discloses machines for the cleaning, sanifying and disinfection of the articles in question which make the task of emptying and cleaning these articles easier and safer for the hospital attendants. These machines make the sanitary auxiliary articles hygienically safer so that they can be reutilized, the risk of a biological contamination being thus removed.

The said machines usually comprise an emptying and washing basin in which the bed-pans and the like are emptied and washed. The basin is closed through a door which is usually provided with positioning and supporting means which position and support the bed-pan and the like during the sanifying process.

However, there is a problem in the utilization of the said machines because every country adopts different auxiliary sanitary articles showing different shapes in order to adapt them to specific uses or particular stocking means or for other reasons.

In particular, there is a type of bed-pan, the so-called "slipper" bed-pan, which is shaped like a slipper. This shape is adopted to facilitate the carrying of the auxiliary sanitary articles when they contain organic rejections because the slipper shape makes it possible to incline the ben-pan so that the contained material slides in the pocket of the slipper bed-pan and remains stabler when the hospital attendant carries the bed-pan.

However, the particular shape of the "slipper" bed-pan involves that it must be inserted in a particular manner in the machine when it is necessary to automatically empty the bed-pan. In practice, the "slipper" bed-pan must be rotated of about 180 degrees in respect to the vertical axis but this movement involves some manual operations which make it necessary to touch the bed-pan (potentially dirty in every part), which increases the risk of contamination or infection for the hospital attendants.

DE-A-36 28 793 discloses a washing - disinfecting machine according to the preamble of claim 1. From that document a device for emptying and cleaning hygiene vessels is known comprising a rinsing chamber having a conical lower part, an odour seal, cleaning nozzles in the rinsing chamber and a horizontal or vertical door.

The aim of the present invention is to facilitate the task far the hospital attendants, nurses and others by permitting a simple insertion of the auxiliary sanitary article in the machine without rotating the bed-pan. In this way, the number of the operations to be performed for the insertion of the bed-pan in the machine are reduced. Consequently, the risk of a biological contamination is reduced when inserting the bed-pan.

An immediate advantage of the present invention consists in a reduction of the operations to be performed to insert the auxiliary sanitary article, in particular a slipper-shaped bed-pan, in the machine, which is made possible by a rotating automatism of the hooking support of the sanitary articles. The rotating automatism arranges the said articles in the correct position automatically before the closure of the washing chamber and the beginning of the treatment.

All the above indicated aims and advantages are reached according to the present invention by a washing-disinfecting machine comprising a hooking support for sanitary articles such as bed-pans, urinals and the like, the washing-disinfecting machine further comprising a washing chamber in which the sanitary articles can be washed and disinfected, an opening through which the sanitary articles to be treated can be introduced, and a door which closes the opening, characterized in that the washing-disinfecting machine comprises an automatic rotating mechanism for the hooking support, wherein the automatic rotating mechanism is fixed to an inner side of the door to rotate the hooking support, the automatic rotating mechanism including a jack or actuator, a toothed bar and a toothed wheel, wherein the toothed bar is fixed to an end of the jack and transmits the motion from the jack or actuator to the toothed wheel the axis of which passes through a hole in the door and supports the hooking support for the sanitary articles to be treated.

The said hooking support or anchoring systems may be interchanged by means of suitable tools so that they can support different types of auxiliary sanitary articles to face the different situations.

The rotating motion of the hooking support which serves to support bed-pans or urinals permits them to be inserted in a normal horizontal position before closing the washing chamber and starting the treatment. In this way, it is possible to avoid a dispersion of the contained rejections. On closing the door the sanitary articles are rotated in such a way as to be overturned, emptied and washed.

Further features and details of the present invention will be better understood from the following specification which is given as an example which does not limit the invention itself on the basis of the accompanying drawings wherein:
- Fig. 1 is a schematic lateral view of a machine for emptying and washing auxiliary sanitary articles, its door being provided with moving means for the hooking supports according to the invention and being open in a first operative phase;
- Fig. 2 is a schematic view of the machine in Fig. 1 in which the door is open in a second operative phase;
- Fig. 3 is a schematic view of the machine in Fig. 2 in which the door is closed in a third operative washing phase;
- Fig. 4 is a schematic front view of the washing machine;

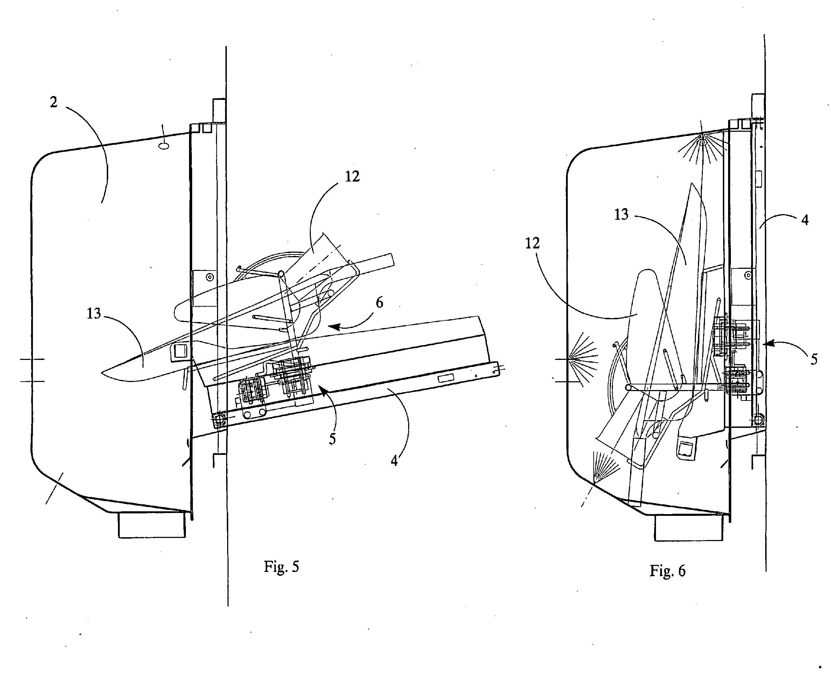

- Figg. 5 and 6 are schematic lateral detailed views of the door in an open and closed position, respectively;

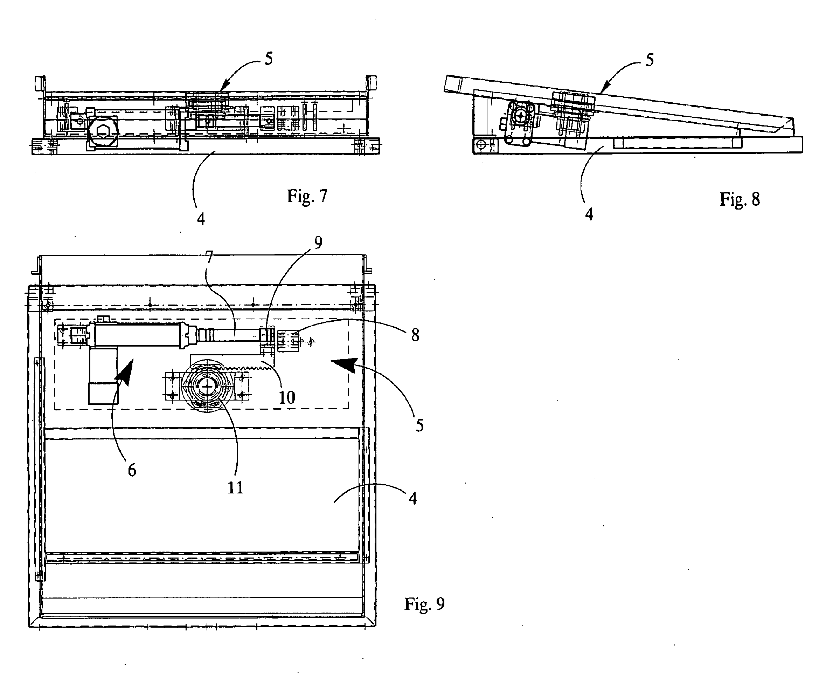

- Figg. 7-9 are schematic front lateral plan views of door and components according to the present invention;
- Figg. 10-15 are schematic views showing the operative phases of the device according to the present invention.

With reference to the accompanying drawings, number 1 denotes a machine as a whole for emptying and washing hospital auxiliary sanitary articles such as bed-pans, urinals and the like. This machine includes typically a washing basin 2 in which the sanitary articles to be emptied and washed are arranged in such a way as to permit the rejections to flow down into discharge ducts 3.

The washing machine 1 is usually provided with a front door 4. Particular connections are arranged on the inner side of the door. The supports for the sanitary articles to be treated are fixed to the said connections.

The peculiarity of the present invention is a system of movement 5 for the said supports which permits the sanitary articles in question, in particular slipper-shaped bed-pans, to be inserted in the washing machine in a normal horizontal position. Only when the door of the washing machine is being closed, the sanitary articles rotate automatically till reaching a vertical position in which the articles are emptied and washed. In this way, the handling of the sanitary articles by the hospital attendants is limited as much as possible.

More precisely, there is provided a mechanism of automatic rotation, indicated on the whole with 6, for a hooking support of bed-pans and urinals. This mechanism is fixed to the inner side of a door 4 of a washing machine and is equipped with a jack or actuator 7. The jack or actuator is fixed to the inner side of the door 4 by means of a blocking unit.

The axial movement of the jack is permitted by a centring rod stop 8. Two rods slide within the stop. One of the two rods is fixed to the jack in order to eliminate or at least to reduce the non-axial movements.

At one end of the jack or actuator there is fixed a head block 9 to which a second centring rod is fixed to eliminate the rotations of the jack rod around the sliding axis.

In addition, a toothed bar 10 is fixed to the head block and transmits the motion from the actuator to a toothed wheel 11 to turn the motion of translation of the jack or actuator rod into a motion of rotation of the toothed wheel 11 around its axis of rotation.

The toothed wheel 11 is fixed through a washer to a blocking element.

The washer makes the toothed wheel integral with the particular mounting of the toothed wheel without preventing or reducing the rotation of the toothed wheel.

An end of the toothed wheel projects in the inner part from the door of the basin through a hole obtained in the inner side of the door to transmit the motion out of the basin door.

Suitable anchoring systems or hooking supports for the sanitary articles to be treated will be fixed to the said end. The anchoring systems or hooking supports are interchangeable by means of suitable tools so that they may support different types of auxiliary tools as occasion may require.

The rotating motion of the toothed wheel is thus transmitted to the hooking support to permit the automatic moving mechanism of the hooking support for the sanitary articles to rotate on bringing the sanitary articles from a horizontal position of insertion to a reversed position of discharge and washing.

In addition, a sealing system of components is provided to prevent the washing liquid and disinfecting vapour from penetrating in the inside of the door of the washing machine and reaching the parts of the automatic moviment mechanism.

The said sealing system of components comprises a screw collar which is positioned in the inside of the door of the washing machine and is fixed to a sealing ring. The union between the screw collar and the sealing ring is permitted by a hole of the inner side of the door of the washing machine.

The plate of the inner door is folded between the two components of the said sealing system. In practice, the two components are fixed to the inner door and can not move.

A sealing ring gasket is arranged between the plate of the inner door and the screw collar to prevent liquid and vapour from getting in the inside of the inner door through interstices of the sealing system.

In turn, a suitable ring gasket is housed in the box of the sealing ring. The ring gasket adheres perfectly to the outer cylindrical surface of the toothed wheel and prevents liquid and vapour from getting in the inside of the door of the washing machine.

Now, an example of the functioning of the device according to the present invention will be described briefly.

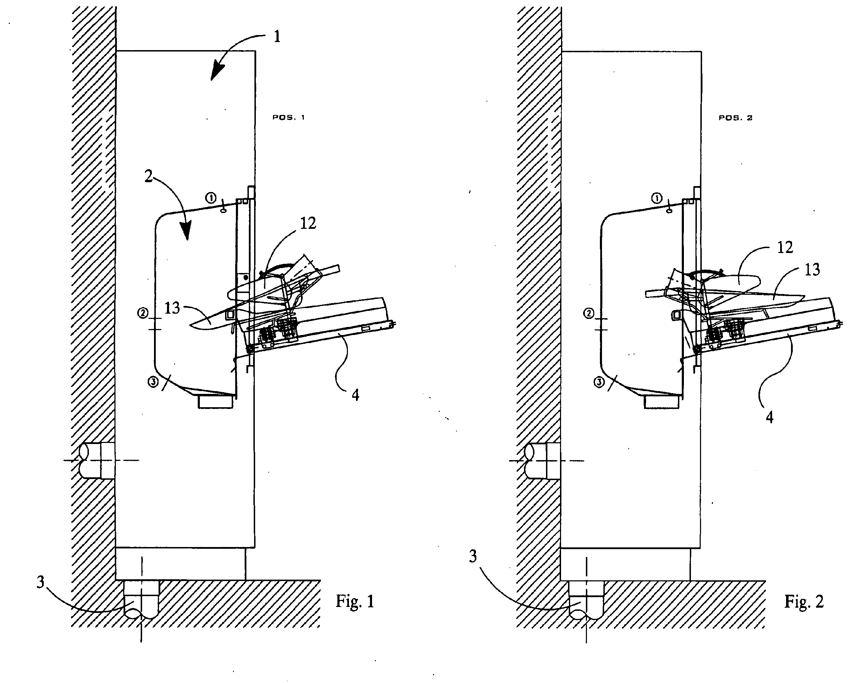

In Figure 1, Position 1, a lateral view of the washing and disinfecting machine 1 is shown. In this view, the door 4 of the washing chamber 2 is open. The automatic moving mechanism for the hooking support of bed-pans and urinals is positioned on the door itself. The hooking support for the bed-pans and urinals is fixed to the automatic moving mechanism.

In addition, from Fig. 1 it appears the position of two hospital auxiliary sanitary articles, namely, an urinal 12 and a slipper-shaped bed-pan 13. The articles 12, 13 are represented just after being inserted in the anchoring system by the hospital attendants or other persons.

The organic material is collected in the left end of the bed-pan where there is the pocket of the bed-pan so that the organic material is prevented from getting out of the bed-pan. The urinal is shaped nearly like a bottle and therefore, the rotation for inserting this sanitary article is simpler than the rotation needed to insert the bed-pan. It is not necessary to touch the urinal in many points.

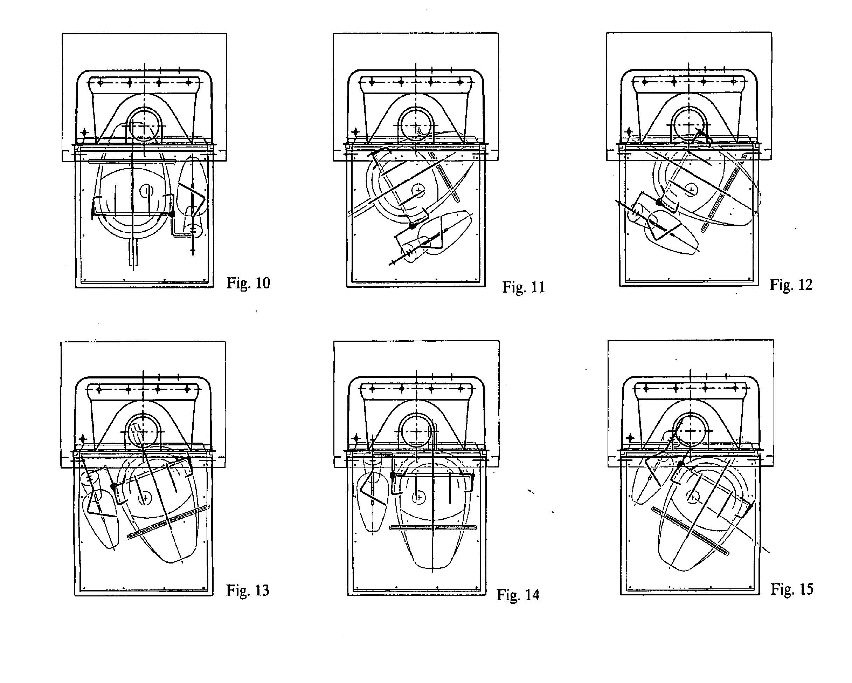

Figure 10 (Drawing 5) is a plan view of the so-described phase from which it appears the position of the sanitary articles in respect to the door.

After the auxiliary sanitary article has been positioned on the hooking support, a suitable washing program is selected depending on the type of sanitary article to be treated and type of material contained in the article. It is sufficient to push one of the buttons to start the necessary washing program.

Firstly, the automatic moving mechanism rotates the hooking support to correctly position the auxiliary articles in the inside of the machine. The rotation is represented in Figures 11-15.

In the said position, the organic material is in the right end of the bed-pan (Fig. 14) in the pocket of the slipper-shaped bed-pan.

When the bed-pan reaches the position represented in Fig. 15, the door begins closing till reaching its vertical position so that owing to the law of gravitation the organic material slides and falls down from the bed-pan pocket to the washing chamber. The same applies to the organic material contained in an urinal.

The particular position of the hooking support in relation to the door (Fig. 15) permits the organic material contained in the bed-pan to fall down in the basin without sliding on the handle of the bed-pan. In this way, the bed-pan handle remains clean and a soiling of same is avoided.

Once the washing chamber is closed by the door 4, which takes its vertical position, and after a few seconds, the automatic moving mechanism accomplishes a rotation contrary to the preceding rotation in order to take the most suitable position for the bed-pan to be hit by the washing jets.

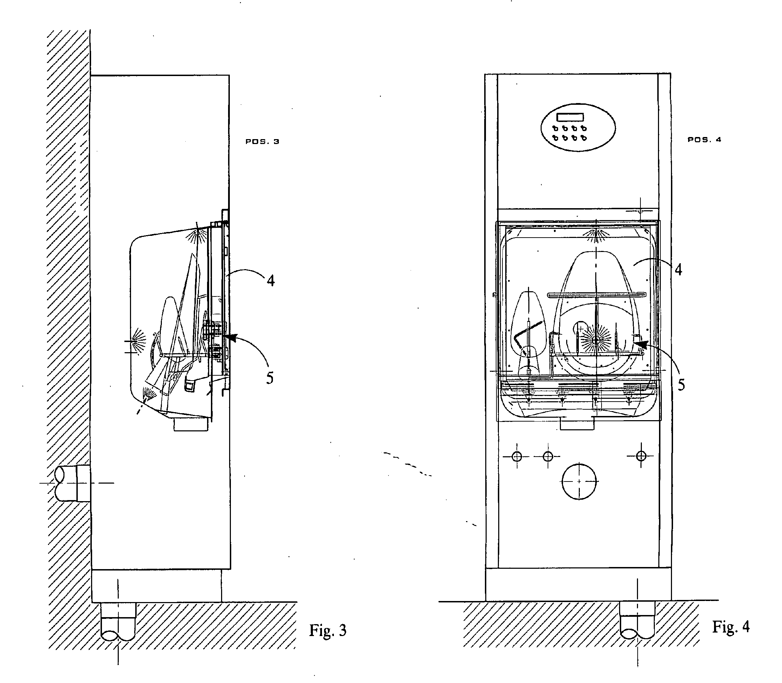

As it appears from Position Figures 1, 2 and 3, an upper washing jet 1 washes the outer surface of the sanitary articles inserted in the washing chamber, a central washing jet 2 washes the inner surface and the contact surface of the bed-pan as well as the urinal put laterally. Finally, a lower washing jet 3 washes the inner surface of the urinal and contributes to the outer and inner washing of the bed-pan.

When the washing cycle is finished, the door is open and returns to the position of Figure 14. At this stage, the automatic moving mechanism for the hooking support of bed-pans rotates automatically in order to bring the whole system back to its initial position of Figure 10 so that the users can take out the washed and disinfected sanitary articles.

Advantageously, it is possible to provide a particular function, the so-called SWING function, which consists in a movement to be automatically accomplished by the automatic moving mechanism during the washing program, which function permits to obtain a better result as regards the removal of dirt from the surfaces of the sanitary articles to be treated.

The initial position of the hooking support in Figure 14 swings through the said function between the position of Figure 13 and the position of Figure 15 The said function consists in the fact that the hooking support shifts from the position of Figure 14 and is swung by the mechanism between the position of Figure 13 and the position of Figure 15 and vice versa. In this way, the hooking support returns to the starting position 2 over and over again so that the water jets can reach more sectors of the sanitary article to be washed in relation to a fixed central position of the hooking support, as represented in Figure 14. The so-described washing operation if thus improved.

As can be seen, the present solution reaches all the above aims and advantages. In particular, the work of the hospital attendants and nurses is made easier and safer because it is sufficient to insert the hospital sanitary articles in question in the washing machine without rotating them. In this way, the number of operations for their insertion is reduced, which avoids the risk of a biological contamination.

The present invention has been described according to a preferred solution but further changes and versions may be considered, for instance regarding the type of mechanical movement for the bed-pan support, in order to obtain solutions to be considered as included in the scope of protection of the present invention as claimed.

## Claims

1. Washing-disinfecting machine comprising a hooking support for sanitary articles such as bed-pans, urinals or the like, the washing-disinfecting machine further comprising a washing chamber in which the sanitary articles can be washed and disinfected, an opening through which the sanitary articles to be treated can be introduced and a door which closes the opening, **characterized in that** the washing-disinfecting machine comprises an automatic rotating mechanism for the hooking support, wherein the automatic rotating mechanism (5) is fixed to an inner side of the door (4) to rotate a the hooking support, the automatic rotating mechanism (5) including a jack or actuator (7), a toothed bar (10), and a toothed wheel (11), wherein the toothed bar (10) is fixed to an end of the jack or actuator (7) and transmits the motion from the jack or actuator to the toothed wheel (11) the axis of which passes through a hole in the door and supports the hooking support for the sanitary articles to be treated.

2. Washing-disinfecting machine as claimed in the foregoing claim, **characterized in that** the jack or actuator (7) is fixed to the door by means of a blocking unit.

3. Washing-disinfecting machine as claimed in the foregoing claims, **characterized in that** the toothed bar (10) turns a translating motion of the jack rod into a rotating motion of the toothed wheel (11) around its axis of rotation.

4. Washing-disinfecting machine as claimed in the foregoing claims, **characterized in that** the hooking supports (6) can be interchanged by means of tools so that different types of auxiliary sanitary articles (12, 13) can be supported.

## Patentansprüche

1. Desinfektionswaschmaschine umfassend einen Trägerhaken für Sanitärartikel wie Bettpfannen, Harnflaschen oder Ähnliches, die Desinfektionswaschmaschine umfasst weiterhin eine Waschkammer, in der die Sanitärartikel gewaschen und desinfiziert werden können, eine Öffnung, durch die die zu behandelnden Sanitärartikeln zugeführt werden können und eine Tür, die Öffnung verschließt, **dadurch gekennzeichnet, dass** die Desinfektionswaschmaschine einen automatischen Drehmechanismus für den Trägerhaken umfasst, wobei der automatische Drehmechanismus (5) an einer Innenseite der Tür (4) angebracht ist, um den Trägerhaken zu drehen, der automatische Drehmechanismus (5) umfasst eine Stellvorrichtung oder einen Aktuator (7), eine Zahnstange (10) und ein Zahnrad (11), wobei die Zahnstange (10) an einem Ende der Stellvorrichtung oder des Aktuators (7) angebracht ist und die Bewegung der Stellvorrichtung oder des Aktuators an das Zahnrad (11) überträgt, dessen Achse eine Ausnehmung in der Tür durchsetzt und den Trägerhaken für die zu behandelnden Sanitärartikel abstützt.

2. Desinfektionswaschmaschine nach dem vorausgegangenen Anspruch, **dadurch gekennzeichnet, dass** die Stellvorrichtung oder der Aktuator (7) an der Tür durch eine Blockiereinheit festgelegt ist.

3. Desinfektionswaschmaschine nach einem der vorausgegangenen Ansprüche, **dadurch gekennzeichnet, dass** die Zahnstange (10) eine Translationsbewegung der Stellvorrichtungsstange in eine Rotationsbewegung des Zahnrads (11) um dessen Rotationsachse umsetzt.

4. Desinfektionswaschmaschine nach einem der vorausgegangenen Ansprüche, **dadurch gekennzeichnet, dass** der Trägerhaken (6) mittels Werkzeug ausgetauscht werden kann, so dass verschiedene Typen unterschiedlicher Sanitärartikel (12, 13) getragen werden können.

## Revendications

1. Machine à laver et à désinfecter comprenant un support de crochetage pour articles d'hygiène tels que des bassins hygiéniques, des urinaux ou analogues, la machine à laver et à désinfecter comprenant en outre une chambre de lavage dans laquelle les articles d'hygiène peuvent être lavés et désinfectés, une ouverture à travers laquelle les articles d'hygiène à traiter peuvent être introduits et une porte qui ferme l'ouverture, **caractérisée en ce que** la machine à laver et à désinfecter comprend un mécanisme de mise en rotation automatique du support de crochetage, le mécanisme de mise en rotation automatique (5) étant fixé à un côté intérieur de la porte (4) pour faire tourner le support de crochetage, le mécanisme de mise en rotation automatique (5) incluant un vérin ou un actionneur (7), une barre dentée (10), et une roue dentée (11), la barre dentée (10) étant fixée à une extrémité du vérin ou de l'actionneur (7) et transmettant le mouvement du vérin ou de l'actionneur à la roue dentée (11) dont l'axe passe à travers le trou ménagé dans la porte et supporte le support de crochetage destinés aux articles d'hygiène à traiter.

2. Machine à laver et à désinfecter selon la revendication précédente, **caractérisée en ce que** le vérin ou l'actionneur (7) est fixé à la porte au moyen d'une unité de blocage.

3. Machine à laver et à désinfecter selon les revendications précédentes, **caractérisée en ce que** la barre dentée (10) convertit un mouvement de translation de la tige de vérin en un mouvement de rotation de la roue dentée (11) autour de son axe de rotation.

4. Machine à laver et à désinfecter selon les revendications précédentes, **caractérisée en ce que** le support de crochetage (6) peut être remplacé au moyen d'outils de sorte que différents types d'articles d'hygiène auxiliaires (12, 13) peuvent être supportés.
